# EUROPEAN PATENT SPECIFICATION

(11) **EP 3 969 076 B1**
(45) Date of publication and mention of the grant of the patent: **29.07.2026**
(21) Application number: 20810250.9
(22) Date of filing: 14.05.2020
(51) Int. Cl.: A61M 5/14, A61M 5/142, A61M 5/145, A61M 5/172

(54) **DRUG INFUSION DEVICE WITH MULTIPLE INFUSION MODES**
MEDIKAMENTENINFUSIONSVORRICHTUNG MIT MEHREREN INFUSIONSMODI
DISPOSITIF DE PERFUSION DE MÉDICAMENT À MULTIPLES MODES DE PERFUSION

(30) Priority: 17.05.2019 WO PCT/CN2019/087342; 01.08.2019 WO PCT/CN2019/098784; 31.12.2019 WO PCT/CN2019/130445; 31.12.2019 WO PCT/CN2019/130442
(43) Date of publication of application: 23.03.2022
(73) Proprietor: Medtrum Technologies Inc., Shanghai 201203 (CN)
(72) Inventor: YANG, Cuijun, Shanghai 201203 (CN)
(74) Representative: Becker, Eberhard
(86) International application number: PCT/CN2020/090147
(87) International publication number: WO 2020/233484

(56) References cited:
- WO-A1-01/70307
- CN-A- 101 137 408
- US-A1- 2003 199 824
- US-A1- 2003 236 498
- US-A1- 2010 094 261
- US-A1- 2011 230 837
- US-A1- 2012 165 734
- US-A1- 2013 253 472
- US-A1- 2019 009 019
- US-A1- 2019 117 881

## Description

### TECHNICAL FIELD

The present invention mainly relates to the field of medical instruments, in particular to a drug infusion device with multiple infusion modes.

### BACKGROUND

A drug infusion device can continuously deliver drug into a patient's body for disease treatment. Drug infusion devices are widely used in the field of diabetes treatment, which continuously infuse required dosage of insulin into the patient's subcutaneous tissue, thereby simulating the secretion function of the pancreas to keep the blood glucose stable. The drug fluid is usually stored inside the infusion pump. The existing drug infusion device, controlled by remote device, is usually attached directly on the patient's skin through a medical adhesive tape. WO 01/70307 A1 relates to an external infusion device that infuses a fluid into an individual's body. US 2013/253472 A1 relates to a method and an apparatus for delivery of a drug to a recipient. US 2019/009019 A1 relates to a device which delivers a fluid medicament to the subcutaneous tissue of a user. US 2011/230837 A1 relates to a system for priming an infusion pump. US 20101094261 A1 relates to a method, a computer program product, and an infusion pump assembly for administering a sequential, multi-part, infusion event.

Currently, the drug infusion device in prior art has single infusion mode, and the user or the system cannot flexibly select the infusion rate or infusion increment of the infusion device with poor user experience.

Therefore, the prior art urgently needs a drug infusion device with multiple infusion modes.

### BRIEF SUMMARY OF THE INVENTION

The invention is set out in the appended set of claims. The following disclosure serves a better understanding of the present invention. The embodiment of the invention discloses a drug infusion device with multiple infusion modes, such as a variety of different drug infusion rates or infusion increments, for the user or system to choose, enhancing the user experience.

The invention discloses a drug infusion device with multiple infusion modes, including: a reservoir, a piston and a screw, the piston, connected with the screw, is arranged in the reservoir; a driving module at least includes a first driving unit and a second driving unit that cooperate with each other, the second driving unit drives the screw forward; a power module connected to the first driving unit; and a control module, connected to the power module, controls the power module to apply different driving powers to the first driving unit, making the first driving unit have a variety of different operating modes, thereby making the infusion device have various different infusion increments or infusion rates.

According to an aspect of the present invention, the operating manner of the first driving unit includes unidirectional movement or reciprocating movement.

According to an aspect of the present invention, the operating mode of the first driving unit includes the amplitude of the unidirectional movement, the amplitude of the reciprocating movement or the movement rate, therefore a variety of different operating modes of the first driving unit include different unidirectional movement or reciprocating movement, or various different movement rates.

According to an aspect of the present invention, the power module applies different forces in linear directions to the first driving unit.

According to an aspect of the present invention, the power module includes a first power unit and a second power unit respectively connected to the first driving unit.

According to an aspect of the present invention, the first power unit includes an electrically driven linear actuator or an electrically heated linear actuator, and the second power unit includes an electrically driven linear actuator, an electrically heated linear actuator or an elastic member, and the control module, through controlling the magnitude or output frequency of the driving power output by the first power unit or the second power unit, controls the amplitude of the unidirectional movement, the amplitude of the reciprocating movement or the movement rate of the first driving unit.

According to an aspect of the present invention, the first driving unit includes at least one driving end, and the second driving unit includes at least one driving wheel provided with wheel teeth, and the driving end pushes the wheel teeth forward to rotate the driving wheel.

According to an aspect of the present invention, the first power unit is an advancing member, while the second power unit is a reset member, and during operation, the advancing member applies driving power to the first driving unit to drive the driving end to advance the wheel teeth, while the reset member applies driving power to the first driving unit to reset the driving end.

According to an aspect of the invention, the driving wheel is a ratchet wheel and the wheel teeth are ratchet teeth.

According to an aspect of the present invention, the driving end includes a linearly reciprocating pawl which pushes the ratchet teeth to rotate the ratchet intermittently.

According to an aspect of the present invention, the driving module further includes at least one rotating shaft, the first driving unit includes at least one driving member provided with the driving end, and during operation, the driving member reciprocatingly rotates around the rotating shaft, driving the driving end to reciprocate to make the driving wheel rotate intermittently.

According to an aspect of the present invention, the driving member includes two driving ends which cooperate with the same driving wheel.

According to an aspect of the present invention, the driving module further includes a rotating shaft, the first driving unit includes at least two driving ends, and the second driving unit includes two fixedly connected driving wheels, and each driving wheel cooperates with at least one driving end.

According to an aspect of the present invention, the first driving unit is disposed between two driving wheels, and during operation, the first power unit and the second power unit alternately apply driving power to the first driving unit, making the first driving unit, in the two direction of its reciprocating rotation, drive different driving wheels to rotate intermittently.

According to an aspect of the present invention, when one driving wheel cooperates with two driving ends, the front ends of the two driving ends are not level, making the two driving ends push the wheel teeth asynchronously.

According to an aspect of the present invention, the driving module further includes at least one auxiliary driving unit which is connected to and cooperated with the first driving unit or the second driving unit, and the first driving unit and the second driving unit cooperate with each other through the auxiliary driving unit.

According to an aspect of the present invention, it further includes a base on which the second driving unit is movably assembled, and the base and the second driving unit are frictional fit.

According to an aspect of the present invention, it further includes a position limited member which is movably assembled on the base to limit the position of the second driving unit, and the position limited member and the second driving unit are frictional fit.

Compared with the prior art, the technical solution of the present invention has the following advantages:
In the drug infusion device with multiple infusion modes disclosed in the present invention, the control module controls the power module to apply different driving powers to the first driving unit, making the first driving unit have a variety of different operating modes, and thereby making the infusion device have various different infusion increments or infusion rates. When the infusion device has a variety of different infusion increments or infusion rates, the user or closed-loop system can arbitrarily choose the appropriate infusion mode to accurately control the level of body fluids according to the actual requirements of the body, improving the user experience.

Furthermore, the power module includes a first power unit and a second power unit respectively connected to the first driving unit. These two power units respectively apply driving power to the first driving unit, which can facilitate the reciprocating movement of the first driving unit.

Furthermore, the first power unit includes an electrically driven linear actuator or an electrically heated linear actuator, while the second power unit includes an electrically driven linear actuator, an electrically heated linear actuator or an elastic member. The magnitude of the power output by the linear actuator can be controlled by the current, therefore the power output is more stable, thus making the amplitude or rate of movement of the first driving unit more stable and controllable. In addition, when the second power unit is an elastic member, the first driving unit can be automatically reset without consuming electric energy, thereby reducing the power consumption of the infusion device.

Furthermore, when one driving wheel cooperates with two driving ends, the front ends of the two driving ends are not level, making the two driving ends push the wheel teeth asynchronously. One driving wheel cooperates with two driving ends, making the infusion device have more infusion modes, therefore the body fluid level is controlled more precisely, improving the user experience.

Furthermore, the driving module further includes at least one auxiliary driving unit. The auxiliary driving unit is connected to and cooperates with the first driving unit or the second driving unit, and the first driving unit and the second driving unit cooperate with each other through the auxiliary driving unit. Through the transitional adjustment of the auxiliary driving unit, the movement manner or movement mode of the second driving unit will be smoother than that of the first driving unit, thus making the drug infusion process more stable, avoiding the drastic fluctuations in body fluid levels and enhancing the user experience.

Furthermore, the second driving unit is movably assembled on the base, and the base and the second driving unit are frictional fit. The friction fit can increase the frictional force the second driving unit receives during its movement. When the first driving unit does not implement actually driving, the second driving unit stops moving to ensure the accuracy of the drug infusion volume and eliminate potential safety hazards.

### BRIEF DESCRIPTION OF THE DRAWINGS

FIG.1a is a schematic block diagram of a drug infusion device with multiple infusion modes according to an embodiment of the present invention;
FIG.1b - FIG.1c are top views of two drug infusion devices with multiple infusion modes according to an embodiment of the present invention;
FIG.2 is a schematic diagram of an internal structure of an infusion module according to an embodiment of the present invention;
FIG.3 is a partial top view of the driving module and the power module in FIG.2;
FIG.4 is a schematic diagram of the reciprocating rotation amplitude of the driving member according to an embodiment of the present invention;
FIG.5a - FIG.5b are schematic diagrams of frictional fit between a driving wheel and a base or a position limited member according to an embodiment of the present invention;
FIG.6a - FIG.6d are schematic diagrams of a driving member, a rotating shaft, a reset member and an advancing member according to an embodiment of the present invention;
FIG.7a - FIG.7b are schematic diagrams of the power direction of the advancing member and the advancing direction of the screw according to another embodiment of the present invention;
FIG.8 is a schematic diagram of a driving module including two driving members and a rotating shaft according to yet another embodiment of the present invention;
FIG.9 is a schematic view of a driving module including two rotating shafts and two driving members according to yet another embodiment of the present invention;
FIG.10a - FIG.10b are schematic diagrams of two driving ends of a driving member cooperating with two driving wheels according to yet another embodiment of the present invention;
FIG.11a - FIG.11b are schematic diagrams of a driving member including two driving ends disposed up and down according to yet another embodiment of the present invention;
FIG.12a - FIG.12b are schematic structural views of a driving member disposed between two driving wheels according to yet another embodiment of the present invention;
FIG.13 is a schematic diagram of a first driving unit which can linearly reciprocate according to yet another embodiment of the present invention;
FIG.14 is a schematic diagram of a driving module using gear transmission according to yet another embodiment of the present invention;
FIG.15 is a schematic diagram of a driving module including an auxiliary driving unit according to yet another embodiment of the present invention.

### DETAILEDDESCRIPTION

As mentioned above, the prior art drug infusion device only has a single infusion mode, therefore the user or the system cannot flexibly select the infusion mode, making the user experience poor.

It is found through research that the cause of the above problem is that the driving module inside the infusion device has only one operating mode, resulting in the infusion device only one infusion increment or one infusion rate.

In order to solve this problem, the present invention provides a drug infusion device with multiple infusion modes. The driving module has a variety of different operating modes, therefore, the infusion device has a variety of different infusion increments or infusion rates. According to the actual requirements of the body, the user or the system can flexibly select the infusion mode, which enhances the user experience.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other structures.

The following description of the exemplary embodiments is merely illustrative, and is not intended to be in any way limiting the invention and its application or use. The techniques, methods and devices that are known to those of ordinary skill in the art may not be discussed in detail, but such techniques, methods and devices should be considered as part of the specification.

It should be noted that similar reference numerals and letters indicate similar items in the following figures. Therefore, once an item is defined or illustrated in a drawing, it will not be discussed further in following description of the drawings.

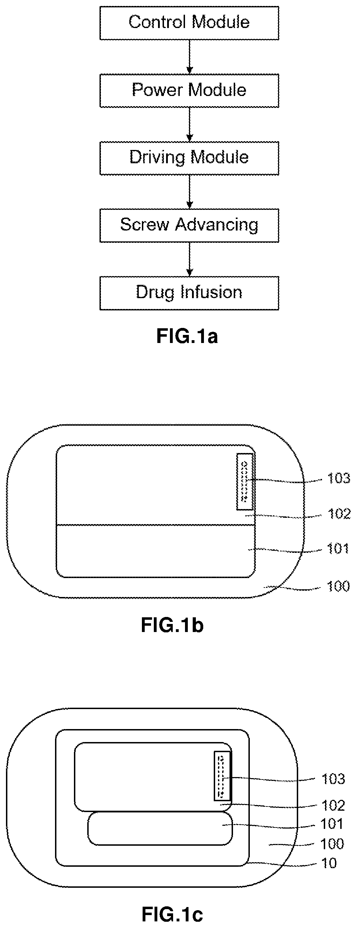

FIG.1a is a schematic diagram of a relationship among modules of a drug infusion device with multiple infusion modes according to an embodiment of the present invention. FIG.1b and FIG.1c are top views of a drug infusion device with multiple infusion modes according to two different embodiments of the present invention, respectively.

As shown in FIG.1a, the control module issues at least an instruction to control the power module to output driving power to the driving module to drive the screw forward, thereby making the infusion device complete drug infusion. For ease of description, the power module, the driving module and the screw are together replaced by infusion module in the following.

Referring to FIG.1b and FIG.1c, the drug infusion device with multiple infusion modes according to the embodiment of the present invention includes: an adhesive patch 100, a control module 101, an infusion module 102, and an infusion needle 103.

The control module 101 is used to control the driving power output by the power module to control drug infusion. The control module 101 may also establish wireless communication with a remote device (not shown). In one embodiment of the present invention, the control module 101 further includes a power supply (not shown).

The infusion module 102 includes various units for achieving the mechanical function of drug infusion, which will be described in detail below in conjunction with different embodiments.

In the embodiment of the present invention, the control module 101 and the infusion module 102 are designed separately and connected by a waterproof plug. The infusion module 102 can be discarded after a single use, while the control module 101 can be reused. In other embodiments of the present invention, the infusion module 102 and the control module 101, connected by a wire, are disposed inside the same housing 10, and both parts will be discarded together after a single use, as shown in FIG.1b.

The adhesive patch 100 is used to attach the infusion module 102 or the control module 101, or both of them as a whole on the skin surface.

One end of the infusion needle 103 is connected to the outlet of the infusion module 102, while the other end pierces the skin to infuse the drug subcutaneously. In the embodiment of the present invention, the infusion needle 103 is provided at one end of the infusion module 102. In other embodiments of the present invention, the infusion needle 103 may also be disposed at other positions according to the functions or the structural features of the device, such as being disposed at the middle portion of the infusion device, which is not specifically limited herein. The infusion needle 103 is a rigid infusion needle or a flexible infusion needle, or according to its different positions and functions, the infusion needle 103 can also adopt a combination of a rigid infusion needle(s) and a flexible infusion needle(s), which is not specifically limited herein. Preferably, in the embodiment of the present invention, the infusion needle 103 is a rigid infusion needle.

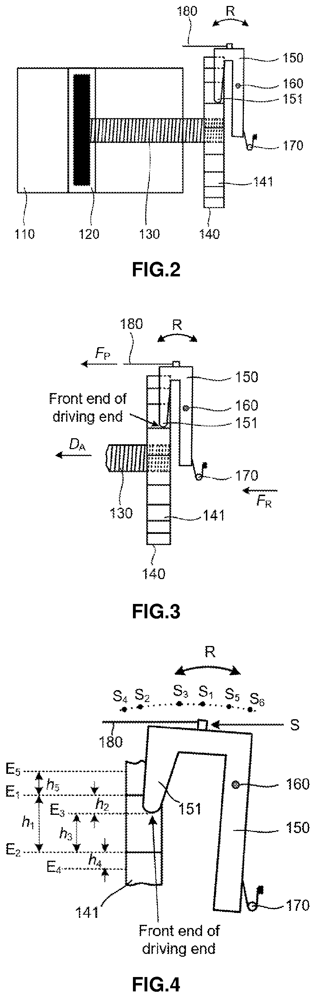

FIG.2 is a schematic diagram of the internal structure of the infusion module 102 according to an embodiment of the present invention.

The internal structure of the infusion module 102 mainly includes a reservoir 110, a piston 120, a screw 130, a driving module and a power module.

The reservoir 110 is used to store drugs which include, but are not limited to, insulin, glucagon, antibiotics, nutrient solutions, analgesics, morphine, anticoagulants, gene therapy drugs, cardiovascular drugs or chemotherapy drugs.

The piston 120 is used to infuse liquid drug into the body.

The screw 130 is connected to the piston 120, thereby pushing the piston 120 to advance, achieving the purpose of drug infusion. The screw 130 is a rigid screw or a flexible screw. When the screw 130 is a flexible screw, the screw 130 may be designed to be curved. In one embodiment of the invention, the flexible screw is formed by a plurality of threaded sub-units movably connected one by one.

The driving module, used to drive the screw 130 forward, includes a first driving unit and a second driving unit that cooperate with each other. The second driving unit is connected to the screw 130. Here, the cooperation means that when the first driving unit operates in a certain manner or mode, the second driving unit will implement an associated operating manner or mode to achieve the goal of driving the screw 130 forward and completing the drug infusion. The meaning of cooperation with each other below is the same as here.

It should to be noted here that the operating manner and operating mode belong to different technical concepts. The operating manner refers to the specific working method or working form, such as unidirectional movement or reciprocating movement, of the first driving unit. However, the operating mode represents the effect, such as the movement amplitude or movement rate, brought about by the operating manner of the first driving unit. The unidirectional movement includes linear unidirectional movement or unidirectional rotation, while the reciprocating movement includes linear reciprocating movement or reciprocating rotation.

The embodiments of the present invention do not limit the types or structural relationships of the first driving unit and the second driving unit, as long as the condition of completing the drug infusion through their cooperation is satisfied. As in one embodiment of the present invention, both the first driving unit and the second driving unit are gears. As in another embodiment of the present invention, the first driving unit is a linear reciprocating pawl, while the second driving unit is a ratchet. As in another embodiment of the present invention, the first driving unit is an airbag, and the second driving unit is a driving rod which can directly pushes the screw 130 forward. And the contraction and relaxation of the airbag drive the driving rod to reciprocate. As in yet another embodiment of the present invention, the driving module further includes one or more auxiliary driving units connected to the first driving unit or the second driving unit, and the first driving unit and the second driving unit may not be in direct contact or connected directly. The auxiliary driving unit transmits the operating manner or operating mode of the first driving unit to the second driving unit, thereby making the second driving unit implement the operating manner or operating mode associated with the first driving unit.

Since the first driving unit is a driving structure and the second driving unit is a driven structure, the power module outputs driving power to the first driving unit which will have a variety of different operating modes, such as different unidirectional movement amplitude, reciprocating movement amplitude or movement rate. And the connection method between the power module and the first driving unit includes mechanical connection or electrical connection.

Specifically, in the embodiment of the present invention, the power module includes a first power unit and a second power unit which are electrically or mechanically connected to and apply driving power to the first driving unit, respectively. The operating mode of the first driving unit includes unidirectional movement or reciprocating movement, which will be described in detail below combined with different embodiments.

The first driving unit includes at least one driving member 150, and the second driving unit includes at least one driving wheel 140 provided with wheel teeth 141. The driving module further includes a rotating shaft 160, and the driving member 150 includes at least one driving end 151. Specifically, in the embodiment of the present invention, the first driving unit is one driving member 150 including only one driving end 151, and the second driving unit is one driving wheel 140, and the first power unit is an advancing member 180 while the second power unit is a reset member 170, as shown in FIG.2. Preferably, for being pushed more easily, the driving wheel 140 is a ratchet with ratchet teeth.

The reset member 170 includes an electrically driven linear actuator, an electrically heated linear actuator, or an elastic member that can automatically reset the driving member 150 without using an external force. The type of elastic members includes, but is not limited to, at least one compression spring, extension spring, torsion spring, elastic sheet, elastic plate, elastic rod, elastic rubber, and the like. Specifically, in the embodiment of the present invention, the reset member 170 is a torsion spring which is more conducive to reset the driving member 150.

In another embodiment of the present invention, the reset member 170 is an electrically driven linear actuator or an electrically heated linear actuator, such as a shape memory alloy. After being energized, the physical form of the material of the linear actuator changes, which makes it shrinkage deformation, thereby outputting driving power to pivot the driving member 150. The higher the current is, the larger shrinkage deformation is, and the greater the driving power outputs. Obviously, when the current is constant, the driving power output by the linear actuator is constant. Therefore, the linear actuator can output a stable and controllable driving power, which makes the infusion process stable and controllable, enhancing the user experience.

The advancing member 180, an electrically driven linear actuator or an electrically heated linear actuator, directly applies driving power to the driving member 150. Specifically, in the embodiment of the present invention, the advancing member 180 is a shape memory alloy.

The control module (not shown) is connected to the power module. In the embodiment of the present invention, the control module applies driving power to the advancing member 180, which makes the driving member 150 drive the driving end 151 to advance the wheel teeth 141, pivoting the driving wheel 140, thereby making the infusion device perform drug infusion.

FIG.3 is a partial top view of the driving module and the power module in FIG.2.

The principle of the driving member 150 driving the driving wheel 140 to rotate in the embodiment of the present invention is as follows. When the control module controls the advancing member 180 to pull the driving member 150 by force *F*_{P}, the driving member 150 rotates counter-clockwise around the rotating shaft 160, driving the driving end 151 to push the wheel teeth 141 forward, thereby making the driving wheel 140 rotate, which makes the screw 130 advance in the *D*_{A} direction and makes the infusion device perform drug infusion. At this time, the reset member 170 is an elastic member which builds a gradually increasing elastic force *F*_{R}. When the advancing member 180 stops applying force and under the action of the elastic force *F*_{R}, the driving member 150 rotates clockwise around the rotating shaft 160. And the driving end 151 stops pushing the wheel teeth 141, therefore the driving wheel 140 stops rotating, and the screw 130 stops advancing, so that the infusion device does not proceed drug infusion. The driving end 151 slides and resets on the surface of the wheel teeth 141 until the driving member 150 stops rotating, which makes the driving member 150 complete one reciprocating rotation R. By analogy, the driving member 150 can complete multiple reciprocating rotations. Obviously, when the infusion device of the embodiment of the present invention is in operation, the rotating manner of the driving wheel 140 is intermittent rotation, that is, a manner of rotation-stop-rotation-stop -.... The meaning of intermittent rotation below is the same as here.

FIG.4 is a schematic diagram of the reciprocating rotation amplitude of the driving member 150 according to an embodiment of the present invention.

Referring to FIG.4, the principle of the driving member 150 implementing two reciprocating rotation amplitudes according to the embodiment of the present invention is as follows. The control module controls the magnitude of the force output of the advancing member 180, and the reset member 170 implements resetting function, which makes the driving member 150 to reciprocate and makes the driving end 151 advance and reset. Eₙ represents the position reached by the front end of the driving end, such as E₁, E₂, E₃, E₄, E₅. *h*ₙ represents the distance between two different positions Eₙ. Sₙ represents the different positions of the point S of the force output by the advancing member 180 during the reciprocating rotation, and the dotted arc in FIG.4 represents the trajectory of S, therefore, S₁, S₂, S₃, S₄, S₅ corresponds with E₁, E₂, E₃, E₄, E₅, respectively. Obviously, the movement distance between different Sₙ can be used to represent the rotation amplitude of the driving member 150. Specifically, in the embodiment of the present invention, h₁ is the pitch of gear tooth, and *h*₁ = 3*h*₂. When the advancing member 180, according to the instruction, makes the driving end 151 to advance the wheel teeth 141 from the E₁ to the E₂ position, the advancing member 180 stops outputting power, and the reset member 170 starts to work until resetting the driving end 151 to the E₃ position, which makes the driving member 150 complete the first reciprocating. The rotation amplitude of the driving member 150 is S₁-S₂ and S₂-S₃. During the first reciprocating rotation, the front end of the driving end pushes a tooth forward by a distance *h*₁, the drug infusion volume is V₁, and its reset distance is *h*₃. At this time, the infusion volume V₁ is regarded as the infusion increment in this first mode. When the next driving is performed, the advancing member 180 outputs force again. During the advancing distance *h*₃ of the driving end, the driving wheel 140 does not rotate, nor the drug infusion of the infusion device. When the front end of the driving end reaches the E₂ position and continues to advance by a distance of *h*₄, the front end of the driving end pushes the wheel teeth 141 to the E₄ position, the driving wheel 140 rotates, implementing the drug infusion. When the advancing member 180 stops outputting the force, the reset member 170 resets the driving end 151 to a certain position, such as the E₅ position, therefore, the driving member 150 completes the second reciprocating rotation, and the driving member 150 rotates by S₃-S₄ and S₄-S₅. During the second reciprocating rotation, the forward distance of the front end of the driving end is (*h*₃ + *h*₄), and the drug infusion volume is V₂. At this time, the infusion volume V₂ is the infusion increment in this second mode. Obviously, the driving member 150 only drives the driving wheel 140 to rotate under the rotation amplitudes S₁-S₂ and S₂-S₄ in these two modes. For the rotation amplitude S₁-S₂ is greater than the rotation amplitude S₂-S₄ (or *h*₁>*h*₄), V₁> V₂. Therefore, the infusion device of the embodiment of the present invention has two different infusion increments.

By analogy, the distance between E₁, E₂, E₃, E₄, E₅ can be arbitrarily selected, such as *h*₁ = *h*₂, *h*₁ = 2*h*₂, *h*₁ = 4*h*₂, etc., the infusion device has a variety of different infusion increments. Or the force point S can also reaches to the S₆ position, and S₄ and S₆ may not be the limit positions for the rotating of the driving member 150, which is not specifically limited herein.

It should be noted that, as described above, in the embodiment of the present invention, the infusion device does not necessarily implement drug infusion when the driving end 151 advances. Only when the driving end 151 pushes the wheel teeth 141 forward, the infusion device does.

Each rotation amplitude of the driving member 150 corresponds with an infusion increment. Therefore, a variety of different rotation amplitudes of the driving member 150 make the drug infusion device have a variety of different infusion increments. Taking insulin as an example, the infusion increment range of the drug infusion device in the embodiment of the present invention is 0.0005U ~ 0.25U (here, the infusion increment range includes endpoint values, that is, the infusion increment includes 0.0005U and 0.25U). In some embodiments of the present invention, the infusion increment of the drug infusion device may includes 0.001U, 0.0025U, 0.005U, 0.0075U, 0.01U, 0.025U, 0.05U, 0.075U, 0.1U, etc. Specifically, in the embodiment of the present invention, the infusion increment of the drug infusion device includes 0.005U, 0.0075U, 0.01U, 0.025U, and 0.05U.

It should be noted here that when *h*₁ = *h*₂, the infusion increment of the infusion device always maintains V₁ with the rotation amplitude always maintaining S₁-S₂ and S₂-S₁, which makes the infusion relatively stable.

Another embodiment of the present invention can also increase the frequency of the force output by the advancing member 180 to increase the frequency of the reciprocating rotation of the driving member 150, thereby increasing the infusion rate of the infusion device. Therefore, the infusion devices in the embodiments of the present invention can all change the power output frequency of the power module to make them have multiple infusion rates. Here, the change of the power output frequency can change the rate of the unidirectional movement, the frequency of intermittent movement, the rate of any single movement, the rate of reciprocating movement, or the frequency of reciprocating movement, which will be described in detail below.

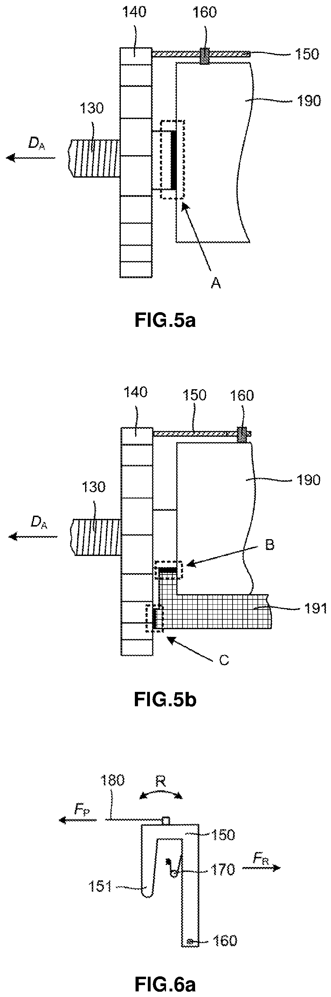

FIG.5a and FIG.5b are schematic diagrams of the driving wheel 140 and the base 190 or the position limited member 191 according to an embodiment of the present invention. FIG.5a and FIG.5b are front views of FIG.3.

The movement of the second driving unit can directly drive the screw forward to complete the drug infusion. Therefore, when the first driving unit does not actually drive, the second driving unit should stop moving. As in the embodiment of the present invention, when the driving end 151 slides on the surface of the wheel teeth 141, the driving end 151, contact with the wheel teeth 141, applies a certain pressure to the driving wheel 140 to ensure the non-rotating of the driving wheel 140. However, it is obvious that due to the structural features of the wheel teeth 141 and the circumference of the driving wheel 140, the pressure applied by the driving end 151 is not equal at different positions. Therefore, when the driving end 151 slides (reset movement or sliding forward) on the surface of the wheel teeth 141, the driving wheel 140 may rotate forward or reverse, which affects the accuracy of the drug infusion volume and brings safety risk.

In an embodiment of the present invention, the second driving unit is movably assembled on the base 190 remaining in frictional engagement. Here, the friction fit means a certain friction force preset between two mutually moving structures, so as to the meaning of the following friction fit. In another embodiment of the present invention, the infusion device further includes a position limited member movably assembled on the base 190 to limit the position of the second driving unit in a frictional engagement way.

As shown in FIG.5a, the driving wheel 140 is movably assembled on the base 190 remaining in frictional engagement. In the embodiment of the present invention, the frictional force of the relative movement between the driving wheel 140 and the base 190 is applied or increased at the position A, indicated by the dotted frame to ensure that when the driving end 151 slides on the surface of the wheel teeth 141, the driving wheel 140 stops rotating.

As shown in FIG.5b, in another embodiment of the present invention, the infusion device further includes a position limited member 191 that is movably assembled on the base 190 to limit the position of the driving wheel 140. The position limited member 191 is in friction fit with the driving wheel 140 at position B or position C, as indicated by the dotted frame. Similarly, in the embodiment of the present invention, the position limited member 191 increases the frictional force that the driving wheel 140 receives when rotating, also ensuring that the driving wheel 140 stops rotating when the driving end 151 slides on the surface of the wheel teeth 141.

Other embodiments of the present invention do not limit the position of the above friction fit, as long as the condition for increasing or applying the friction force received by the second driving unit during movement is satisfied. For example, the friction force can also be applied on both sides of the driving wheel 140 at the same time. The embodiment of the present invention neither limits the material of the position limited member 191. For example, the position limited member 191 is an elastic member, a plastic member or a metal member.

Other embodiments of the present invention may increase the pressure of the driving end 151 on the wheel teeth 141 instead of providing the above-mentioned friction fit, which can increase the maximum static friction of the driving wheel 140 and ensure the non-rotating of the driving wheel 140 when the driving end 151 slides on the surface of the wheel teeth 141.

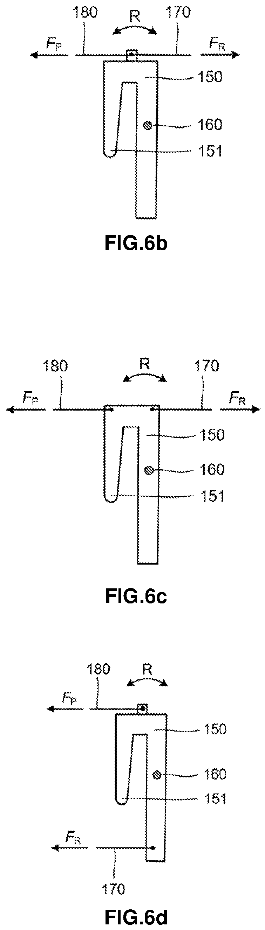

FIG.6a - FIG.6d are schematic diagrams of the driving member 150, the rotating shaft 160, the reset member 170, and the advancing member 180 according to other embodiments of the present invention.

As described above, the power module outputs different forces on the first driving unit to make the first driving unit have multiple different operating modes. Since the force is a vector, different force means different magnitude or direction, or that the force is applied at different positions of the first driving unit. In the embodiment of the present invention, the power module applies force in different linear directions to the first driving unit. In FIG.6b - FIG.6d, the reset member 170 is a shape memory alloy. Obviously, the *F*ₚ direction is parallel to the *F*_{R} and *D*_{A} direction. Such a parallel design can make full use of the space and structural relationship inside the device, making the internal structure more compact.

It should be noted here that because the shape memory alloy is inelastic, the driving member 150 cannot be automatically reset after stopping the advance only in the case that the reset member 170 is energized by the control module to build power to reset the driving member 150.

Since the force can change the movement state of the first driving unit, in other embodiments of the present invention, the power module can also apply a force that makes the first driving unit to have different rotation rates or different rotation modes, which will be described in detail below.

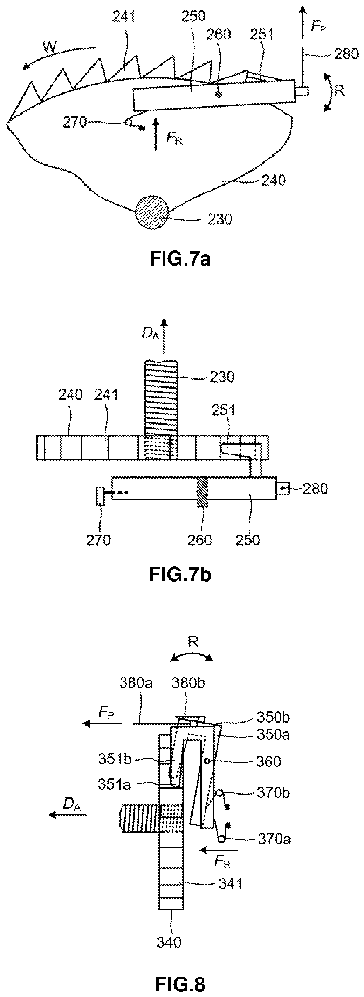

FIG.7a and FIG.7b are schematic diagrams of the structure in which the force *F*_{P} direction of the advancing member 280 is not parallel to the advancing direction *D*_{A} of the screw 230 according to an embodiment of the present invention.

The *F*_{P} direction may not be parallel with the *F*_{R} direction, which is not specifically limited here, as long as the purpose of reciprocating rotation of the driving member 250 can be achieved. As shown in FIG.7a and FIG.7b, the direction of the pulling force *F*_{P} of the advancing member 280 is perpendicular to the advancing direction *D*_{A} of the screw 230. The rotating shaft 260 and the reset member 270 are provided on a base (not shown). As described above, the driving member 250 reciprocally rotates in the R direction to drive the driving end 251 to push the wheel teeth 241, rotating the driving wheel 240 in the W direction, thereby driving the screw 230 to advance in the *D*_{A} direction. The working principle and operating mode of the driving member 250 are the same as described above.

FIG.8 is a schematic diagram of a driving module including two driving members and a rotating shaft 360 according to another embodiment of the present invention. The two driving members share the same rotating shaft 360.

As shown in FIG.8, the two driving members 350a and 350b can independently operate around the rotating shaft 360, which means that each driving member 350a and 350b can independently push the wheel teeth 341 forward. The independent working principle and operating mode of the driving member 350a or 350b are the same as that described above.

The control module of the embodiment of the present invention can also control the power output of the advancing members 380a and 380b, and combined with the reset members 370a and 370b, they can make the driving ends 351a and 351b alternately push the wheel teeth 341 forward, thereby rotating the driving wheel 340 to implement the drug infusion.

In the embodiment of the present invention, as long as a certain driving end reaches the driving position, the wheel teeth 341 can be pushed forward. The driving position here refers to the position where the driving end can, but not necessarily, push the wheel teeth forward, as shown in the positions E₁ and E₂ in FIG.4, and the following driving position has the same meaning as here.

In operation, by controlling the rotation amplitude of the driving members 350a and 350b, the driving ends 351a and 351b alternately push the wheel teeth 341 forward, thereby enabling the infusion device to have a variety of different infusion increments.

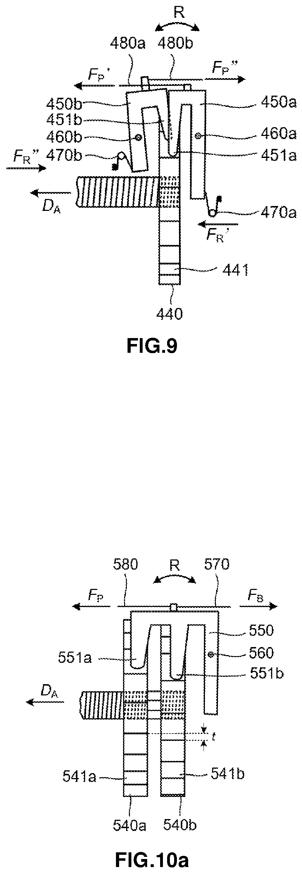

FIG.9 is a schematic diagram of a driving module including two rotating shafts and two driving members according to yet another embodiment of the present invention.

As shown in FIG.9, the two driving members 450a and 450b reciprocally rotate around the rotating shafts 460a and 460b, respectively. The two driving members 450a and 450b and the rotating shafts 460a and 460b are respectively disposed on both sides of the driving wheel 440. The driving members 450a and 450b reciprocally rotate under the force of *F*_{P}', *F*_{R}' and the force of *F*_{P}", *F*_{R}", respectively, to drive the driving ends 451a and 451b forward or reset. The driving members 450a or 450b have different rotation amplitudes, and the driving members 450a or 450b can also independently push the wheel teeth 441 following working principles and operating modes described above. Similarly, the driving members 450a and 450b can also cooperate with each other to alternately push the wheel teeth 441 forward.

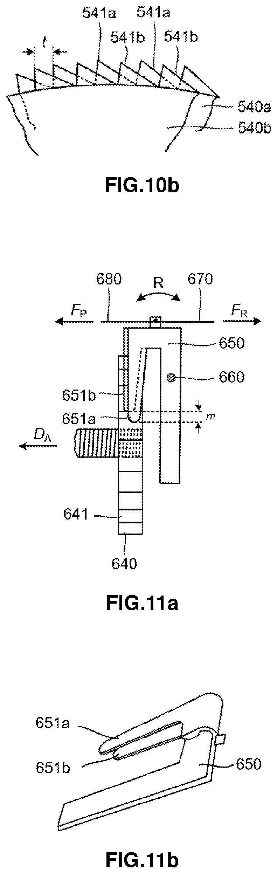

FIG.10a and FIG.10b are schematic diagrams of two driving ends 551a and 551b of a driving member 550 cooperating with two driving wheels 540a and 540b respectively according to yet another embodiment of the present invention. FIG.10b is a right side view of the partial wheel teeth structure of the driving wheels 540a and 540b in FIG.10a.

As shown in FIG.10a and FIG.10b, in the embodiment of the present invention, the driving member 550 includes two driving ends 551a and 551b disposed left and right, while the second driving unit includes two fixedly connected driving wheels 540a and 540b also disposed on the left and right(that is, two driving wheels can move simultaneously). The driving ends 551a and 551b cooperate with the driving wheels 540a and 540b, respectively, and the rotating shaft 560 is disposed on the same side of two driving wheels 540a and 540b. Both the advancing member 580 and the reset member 570 of the embodiment of the present invention are shape memory alloys, and the driving end 551a or 551b can respectively push the wheel teeth 541a or 541b forward. Their working principles and operating modes are consistent with the foregoing.

In addition to driving end 551a or 551b operating independently, the embodiment of the present invention can also adjust the distance between the front ends of the driving ends 551a and 551b, or adjust the offset degree of the wheel teeth 541a and 541b to make two driving ends 551a and 551b cooperate with each other. Preferably, in the embodiment of the present invention, the wheel teeth 541a and 541b are offset with degree t, as shown in FIG.10a and FIG.10b. The following teeth offset of two driving wheels have the same meaning here.

Obviously, in the embodiment of the present invention, two driving ends 551a and 551b reciprocate synchronously. As shown in FIG.10a, when the previous forward movement is completed, the driving member 550 starts a reset movement, the driving end 551a reaches the driving position before the driving end 551b, so the driving end 551a can be used to start the next forward movement instead. Or the driving member 550 continues the reset movement until the driving end 551b reaches the next driving position to start the next forward movement. Of course, the driving member 550 may continue to be reset for a much larger distance, as described above.

Therefore, by controlling the rotation amplitude of the driving member 550, the driving end 551a or 551b can individually push the corresponding wheel teeth 541a or 541b forward, or the driving end 551a or 551b alternately pushes the wheel teeth forward, making the infusion device have multiple infusion increments.

FIG.11a and FIG.11b are still another embodiment of the present invention in which the driving member 650 includes two driving ends 651a and 651b disposed up and down, and driving ends 651a and 651b cooperate with the same driving wheel 640. FIG.11b is a perspective diagram of the driving member 650 in FIG.11a.

As shown in FIG.11a and FIG.11b, the driving member 650 includes two driving ends 651a and 651b disposed up and down cooperating with the same driving wheel 640, so the driving ends 651a and 651b reciprocate synchronously. The front ends of the driving ends 651a and 651b are not level with a certain distance m, therefore, the two cannot simultaneously push the wheel teeth 641 forward, as shown in FIG.11a. When the driving end 651b finishes the last forward movement, the driving member 650 performs a reset movement, obviously making the driving end 651a reach the next driving position before the driving end 651b. The driving end 651a can be used to push the wheel teeth 641 forward to start the next forward movement. Or the driving member 650 continues the reset movement until the driving end 651b reaches the next driving position to start the next forward movement. Of course, the driving ends 651a and 651b can also reset to a much larger distance, as described above.

Therefore, by controlling the power output by the advancing member 680 or the reset member 670, the driving member 650 has different rotation amplitudes, which makes the driving end 651a or 651b individually push the wheel teeth 641 forward or the two alternately push the wheel teeth 641 forward, thereby making the infusion device have a variety of different infusion increments.

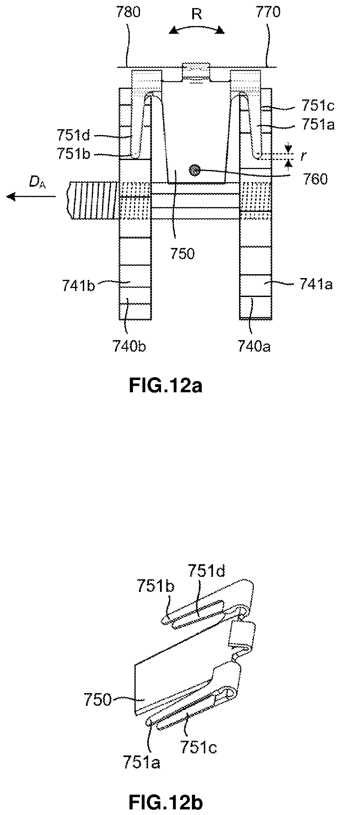

FIG.12a and FIG.12b are schematic diagrams of a driving member 750 disposed between two driving wheels 740a and 740b according to yet another embodiment of the present invention. FIG.12b is a perspective diagram of the driving member 750 in FIG.12a.

As shown in FIG.12a and FIG.12b, the driving member 750 includes four driving ends 751a, 751b, 751c and 751d, and the second driving unit includes two fixedly connected driving wheels 740a and 740b. The driving ends 751a and 751c are disposed on one side of the driving member 750 and cooperate with the driving wheel 740a, while the driving ends 751b and 751d are disposed on the other side to cooperate with the driving wheel 740b. In the embodiment of the present invention, both the first power unit 780 and the second power unit 770, making the driving member 750 reciprocate, are shape memory alloys.

Specifically, in the embodiment of the present invention, the driving member 750 can drive the driving end in both directions of the reciprocating rotation to push the wheel teeth forward for drug infusion. When the driving member 750 rotates clockwise, the driving end 751b or 751d can push the wheel teeth 741b forward, thereby making driving wheels 740a and 740b rotate synchronously and the infusion device perform drug infusion, while driving ends 751a and 751c slide on the surface of the wheel teeth 741a to reset. When the driving member 750 rotates counter-clockwise, the driving end 751a or 751c can push the wheel teeth 741a forward, thereby making the driving wheels 740b and 740a rotate synchronously and the infusion device perform drug infusion, while driving ends 751b and 751d slide on the surface of the wheel teeth 741b to reset.

Similar to the operating mode of the driving ends 651a and 651b described above, in the embodiment of the present invention, the front ends of driving ends 751a and 751c or driving ends 751b and 751d are not level with a distance r. Obviously, in the embodiment of the present invention, as long as there is a driving end in the driving position, this driving end can push the wheel teeth forward for drug infusion. Therefore, by adjusting the offset degree of the teeth on two driving wheels, or adjusting the distance that the driving end advances in each driving process (or that the driving end resets on the other side), the driving member 750 has various rotation amplitudes, making the infusion device have multiple different infusion increments.

In other embodiments of the present invention, there may be two or more driving ends disposed on the driving member 750. When the number of driving ends is an odd number, like 3, 5, etc., the number of driving end(s) that cooperate with each driving wheel is not equal, but each driving wheel works with at least one driving end. The operating mode and working principle of the driving end on each side of the driving member 750 can refer to the foregoing.

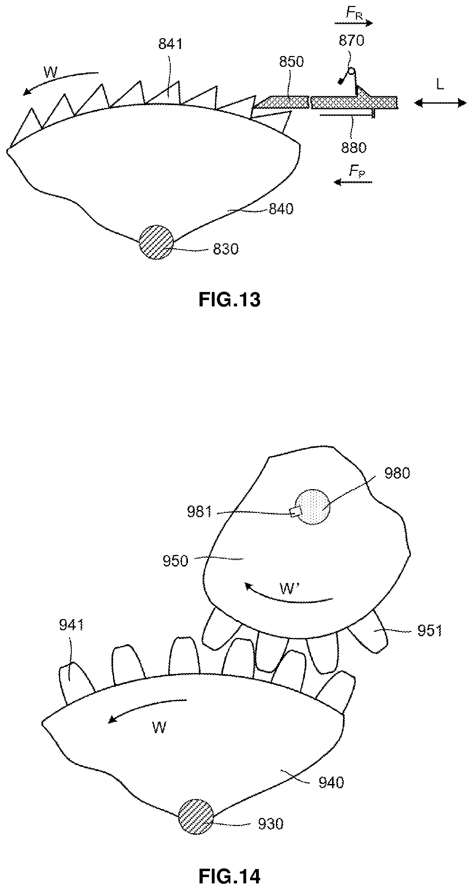

FIG.13 is a schematic diagram of a first driving unit capable of linear reciprocating movement according to yet another embodiment of the present invention.

The first driving unit is a pawl 850 that can linearly reciprocate, while the second driving unit is a ratchet 840 provided with ratchet teeth 841. The power module includes a first power unit 880 and a second power unit 870. The first power unit 880 is a shape memory alloy, and the second power unit 870 is an elastic member.

The first power unit 880 and the second power unit 870 respectively apply force *F*_{P} and *F*_{R} to the pawl 850, making the pawl 850 linearly reciprocate in the L direction. The first power unit 880 can pull the pawl 850 to push the ratchet teeth 841 forward, driving the ratchet 840 to rotate in the W direction and performing drug infusion. The second power unit 870 can reset the pawl 850. Similar to the operating manner or operating mode of the driving end 151 in FIG.4, with the cooperation of the first power unit 880 and the second power unit 870, the amplitude of the forward movement and reset movement of the pawl 850 may change, making the infusion device being equipped with various infusion increments.

It should be noted here that, as described above, the type of the second power unit 870 may have various options, such as a shape memory alloy. Moreover, the embodiment of the present invention does not specifically limit the force applying position and principle of the first power unit 880 and the second power unit 870, as long as the condition that the pawl 850 linearly reciprocates is satisfied.

FIG.14 is a schematic diagram of a gear transmission used in the driving module of another embodiment of the present invention.

As shown in FIG.14, the first driving unit 950 and the second driving unit 940 that cooperate with each other are gears. The power module 980 is fixedly connected to the first driving unit 950 through a pin key 981, therefore they can move synchronously and unidirectionally in the W' direction (here is that the first driving unit 950 rotates unidirectionally), thereby making the second driving unit 940 rotates in the W direction, driving the screw 930 forward and achieving drug infusion.

In other embodiments of the present invention, the power module 980 may also be fixedly connected to the first driving unit 950 in other methods to achieve synchronous movement, such as the cross-sectional shape of the power module 980 is non-circular, thereby ensuring the synchronized movement of the power module 980 and the first driving unit 950 without extra members.

The operating mode of the power module 980 is controlled by the control module. Here, the operating mode of the power module 980 includes the rate of continuous rotation of the power module 980, or the amplitude or frequency of intermittent rotation.

Therefore, when the infusion device of the embodiment of the present invention is in operation, the control module controls the power module 980 to continuously rotate at a number of different and fixed rates, or at a varying rate, making the infusion device have different infusion rates. In another embodiment of the present invention, the power module 980 intermittently rotates with different rotation amplitudes, making the infusion device have multiple different infusion increments. Obviously, the power module 980 can also rotate intermittently at a certain amplitude, which is not specifically limited herein.

In other embodiments of the present invention, one or more auxiliary driving units, such as one or more gears, may also be disposed between the first driving unit 950 and the second driving unit 940. Similarly, through the mutual transmission between multiple gears, the infusion device has a variety of different infusion increments or infusion rates.

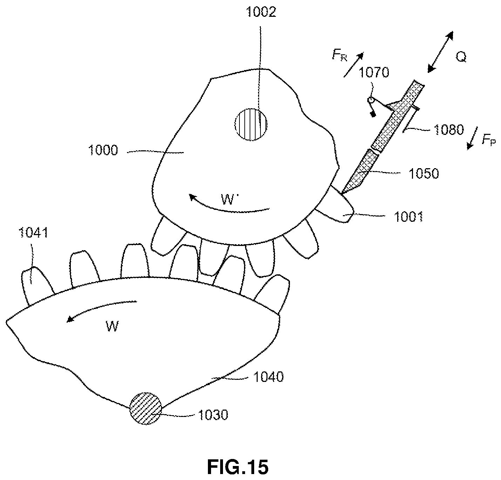

FIG.15 is a schematic diagram of a driving module including an auxiliary driving unit 1000 according to still another embodiment of the present invention.

In the embodiment of the present invention, the auxiliary driving unit 1000 is a gear. The first driving unit 1050 and the second driving unit 1040, cooperating with each other through the auxiliary driving unit 1000, are not in direct contact, which means the auxiliary driving unit 1000 can transmit the operating mode of the first driving unit 1050 to the second driving unit 1040.

Specifically, in the embodiment of the present invention, the first power unit 1080 is a shape memory alloy, while the second power unit 1070 is an elastic member, and the first driving unit 1050 is a driving rod, while the second driving unit 1040 is a driving wheel. Under the cooperation of the first power unit 1080 and the second power unit 1070, the driving rod can linearly reciprocate in the Q direction with the operating mode similarly shown in FIG.13. The auxiliary driving unit 1000 is movably connected to the base (not shown) through the gear shaft 1002. And the FIG.14 shows the operating mode of the auxiliary driving unit 1000 and the second driving unit 1040 cooperating with each other. Therefore, the operating mode of the driving rod is transmitted into the operating mode of the second driving unit 1040 through the auxiliary driving unit 1000, making the infusion device have a variety of different infusion increments or infusion rates.

In the embodiment of the present invention, the auxiliary driving unit can also play a regulating role, which makes the operating process of the second driving unit more stable, thus the infusion process is smoothly controlled. For example, when both the auxiliary driving unit and the second driving unit are gears, by adjusting the diameter ratio (transmission ratio) of them, the second driving unit can operate more smoothly.

When the drug infusion device has multiple infusion modes, the user, according to the actual requirements, can flexibly select the infusion mode to stabilize the level of body fluid parameters. Taking insulin stabilizing blood glucose levels as an example, some users or body tissues at the infusion site absorb insulin slowly. Users can choose a infusion mode with smaller infusion increment or lower infusion rate, which not only stabilizes the blood glucose level, but also improves the utilization of insulin, reducing the burden on body tissues. As another example, blood glucose spikes after a meal, so the user can first select an infusion mode with a relatively large infusion increment or a relatively high infusion rate to suppress the rapid rise in blood glucose, and then select an infusion mode with a medium infusion increment or infusion rate, and finally, choose an infusion mode with a relatively small infusion increment or a relatively low infusion rate to slowly stabilize blood glucose at a reasonable level. For another example, the bolus insulin required after each meal is different, and the body's basal insulin requirement is also different at different periods of one day. Therefore, multiple infusion modes of the infusion device can be flexibly selected (by the user or automatically by the closed-loop system) according to the actual requirements to achieve the goal of precise control of blood glucose levels.

In summary, the drug infusion device with multiple infusion modes disclosed in the present invention has a variety of different infusion increments or infusion rates. According to the actual requirements of the body, the user or the closed-loop system can independently choose different infusions mode to precisely control body fluid levels, improving user experience.

The scope of the invention is defined by the appended claims.

## Claims

1. A drug infusion device with multiple infusion modes, including:
a reservoir (110), a piston (120) and a screw (130), the piston (120), connected with the screw (130), is arranged in the reservoir (110);
a driving module, used to drive the screw (130) forward, at least includes a first driving unit (850, 950) and a second driving unit that cooperate and link with each other, wherein the second driving unit is connected to the screw (130) to drive the screw (130) forward;
a power module (980) connected to the first driving unit; and
a control module (101), connected to the power module (980), controls the power module (980) to apply different driving powers to the first driving unit, making the first driving unit have a variety of different operating modes, **characterized in that**,
the power module (980) includes a first power unit (780, 880, 1080) and a second power unit (770, 870, 1070) respectively connected to the first driving unit,
the first driving unit includes at least one driving member (150, 250, 350a, 350b, 450a, 450b, 550, 650, 750) having at least one rotating shaft (160, 260, 360, 460a, 460b, 560) and at least one driving end (151, 251, 351a, 351b, 451a, 451b, 551a, 551b, 651a, 651b, 751a, 751b, 751c, 751d) rotating along the at least one rotating shaft (160, 260, 360, 460a, 460b, 560), and the second driving unit includes at least one driving wheel (140, 240, 340, 440, 540a, 540b, 640, 740a, 740b) provided with wheel teeth (141, 241, 341, 441, 541a, 541b, 641, 741a, 741b), and the driving end (151, 251, 351a, 351b, 451a, 451b, 551a, 551b, 651a, 651b, 751a, 751b, 751c, 751d) pushes the wheel teeth (141, 241, 341, 441, 541a, 541b, 641, 741a, 741b) forward to rotate the driving wheel (140, 240, 340, 440, 540a, 540b, 640, 740a, 740b), so as to drive the screw (130) forward, thereby making the infusion device provide drug infusion,
the first power unit (780, 880, 1080) is an advancing member (180, 280, 380a, 380b, 580, 680), while the second power unit (770, 870, 1070) is a reset member (170, 270, 370a, 370b, 570, 670), and during operation, the control module (101) controls the advancing member (180, 280, 380a, 380b, 580, 680 to apply driving power to the first driving unit to drive the driving end (151, 251, 351a, 351b, 451a, 451b, 551a, 551b, 651a, 651b, 751a, 751b, 751c, 751d) to advance the wheel teeth (141, 241, 341, 441, 541a, 541b, 641, 741a, 741b), and controls the reset member (170, 270, 370a, 370b, 570, 670) to apply driving power to the first driving unit to reset the driving end (151, 251, 351a, 351b, 451a, 451b, 551a, 551b, 651a, 651b, 751a, 751b, 751c, 751d),
the operating manner of the first driving unit includes unidirectional movement or reciprocating movement,
the operating mode of the first driving unit includes the amplitude of the unidirectional movement, the amplitude of the reciprocating movement or the movement rate, therefore a variety of different operating modes of the first driving unit include different unidirectional movement or reciprocating movement, or various different movement rates,
the power module (980) applies different forces in linear directions to the first driving unit,
the first power unit (780, 880, 1080) includes an electrically driven linear actuator or an electrically heated linear actuator, and the second power unit (770, 870, 1070) includes an electrically driven linear actuator, an electrically heated linear actuator or an elastic member, and the control module (101), through controlling the magnitude or output frequency of the driving power output by the first power unit (780, 880, 1080) or the second power unit (770, 870, 1070), controls the amplitude of the unidirectional movement, the amplitude of the reciprocating movement or the movement rate of the first driving unit,
the at least one driving end (151, 251, 351a, 351b, 451a, 451b, 551a, 551b, 651a, 651b, 751a, 751b, 751c, 751d) includes at least two driving ends (151, 251, 351a, 351b, 451a, 451b, 551a, 551b, 651a, 651b, 751a, 751b, 751c, 751d), and the at least one driving wheel (140, 240, 340, 440, 540a, 540b, 640, 740a, 740b) includes two fixedly connected driving wheels (140, 240, 340, 440, 540a, 540b, 640, 740a, 740b), and each driving wheel (140, 240, 340, 440, 540a, 540b, 640, 740a, 740b) cooperates with at least one of the driving ends (151, 251, 351a, 351b, 451a, 451b, 551a, 551b, 651a, 651b, 751a, 751b, 751c, 751d).

2. A drug infusion device with multiple infusion modes of claim 1, **characterized in that**, the driving wheel (140, 240, 340, 440, 540a, 540b, 640, 740a, 740b) is a ratchet wheel and the wheel teeth (141, 241, 341, 441, 541a, 541b, 641, 741a, 741b) are ratchet teeth.

3. A drug infusion device with multiple infusion modes of claim 2, **characterized in that**, the driving end (151, 251, 351a, 351b, 451a, 451b, 551a, 551b, 651a, 651b, 751a, 751b, 751c, 751d) includes a linearly reciprocating pawl (850) which pushes the ratchet teeth to rotate the ratchet intermittently.

4. A drug infusion device with multiple infusion modes of claim 1, **characterized in that**, the first driving unit is disposed between two driving wheels (140, 240, 340, 440, 540a, 540b, 640, 740a, 740b), and during operation, the first power unit (780, 880, 1080) and the second power unit (770, 870, 1070) alternately apply driving power to the first driving unit, making the first driving unit, in the two direction of its reciprocating rotation, drive different driving wheels (140, 240, 340, 440, 540a, 540b, 640, 740a, 740b) to rotate intermittently.

5. A drug infusion device with multiple infusion modes of claim 1, or claim 4, **characterized in that**,
when one driving wheel (140, 240, 340, 440, 540a, 540b, 640, 740a, 740b) cooperates with two driving ends (151, 251, 351a, 351b, 451a, 451b, 551a, 551b, 651a, 651b, 751a, 751b, 751c, 751d), the front ends of the two driving ends (151, 251, 351a, 351b, 451a, 451b, 551a, 551b, 651a, 651b, 751a, 751b, 751c, 751d) are not level, making the two driving ends (151, 251, 351a, 351b, 451a, 451b, 551a, 551b, 651a, 651b, 751a, 751b, 751c, 751d) push the wheel teeth (141, 241, 341, 441, 541a, 541b, 641, 741a, 741b) asynchronously.

6. A drug infusion device with multiple infusion modes of claim 1, **characterized in that**, the driving module further includes at least one auxiliary driving unit (1000) which is connected to and cooperated with the first driving unit or the second driving unit, and the first driving unit and the second driving unit cooperate with each other through the auxiliary driving unit (1000).

7. A drug infusion device with multiple infusion modes of claim 1, **characterized in that**, it further includes a base (190) on which the second driving unit is movably assembled, and the base (190) and the second driving unit are frictional fit.

8. A drug infusion device with multiple infusion modes of claim 7, **characterized in that**, it further includes a position limited member (191) which is movably assembled on the base (190) to limit the position of the second driving unit, and the position limited member (191) and the second driving unit are frictional fit.

## Patentansprüche

1. Arzneimittelinfusionsvorrichtung mit mehreren Infusionsmodi, umfassend:
ein Reservoir (110), einen Kolben (120) und eine Schraube (130), wobei der mit der Schraube (130) verbundene Kolben (120) in dem Reservoir (110) angeordnet ist;
ein Antriebsmodul, das verwendet wird, um die Schraube (130) vorzuschieben, umfasst mindestens eine erste Antriebseinheit (850, 950) und eine zweite Antriebseinheit, die miteinander zusammenwirken und gekoppelt sind, wobei die zweite Antriebseinheit mit der Schraube (130) verbunden ist, um die Schraube (130) vorzuschieben;
ein mit der ersten Antriebseinheit verbundenes Leistungsmodul (980); und
ein mit dem Leistungsmodul (980) verbundenes Steuermodul (101), das das Leistungsmodul (980) so steuert, dass es unterschiedliche Antriebsleistungen auf die erste Antriebseinheit aufbringt, wodurch die erste Antriebseinheit eine Vielzahl verschiedener Betriebsmodi aufweist, **dadurch gekennzeichnet, dass**
das Leistungsmodul (980) eine erste Leistungseinheit (780, 880, 1080) und eine zweite Leistungseinheit (770, 870, 1070) umfasst, die jeweils mit der ersten Antriebseinheit verbunden sind,
die erste Antriebseinheit mindestens ein Antriebselement (150, 250, 350a, 350b, 450a, 450b, 550, 650, 750) mit mindestens einer Drehwelle (160, 260, 360, 460a, 460b, 560) und mindestens einem Antriebsende (151, 251, 351a, 351b, 451a, 451b, 551a, 551b, 651a, 651b, 751a, 751b, 751c, 751d) umfasst, das sich entlang der mindestens einen Drehwelle (160, 260, 360, 460a, 460b, 560) dreht, und die zweite Antriebseinheit mindestens ein Antriebsrad (140, 240, 340, 440, 540a, 540b, 640, 740a, 740b) umfasst, das mit Radzähnen (141, 241, 341, 441, 541a, 541b, 641, 741a, 741b) versehen ist, und das Antriebsende (151, 251, 351a, 351b, 451a, 451b, 551a, 551b, 651a, 651b, 751a, 751b, 751c, 751d) die Radzähne (141, 241, 341, 441, 541a, 541b, 641, 741a, 741b) vorschiebt, um das Antriebsrad (140, 240, 340, 440, 540a, 540b, 640, 740a, 740b) in Drehung zu versetzen, um die Schraube (130) vorzuschieben, wodurch die Infusionsvorrichtung eine Arzneimittelinfusion bereitstellt;
die erste Leistungseinheit (780, 880, 1080) ein Vorschubelement (180, 280, 380a, 380b, 580, 680) ist, während die zweite Leistungseinheit (770, 870, 1070) ein Rückstellelement (170, 270, 370a, 370b, 570, 670) ist, und während des Betriebs das Steuermodul (101) das Vorschubelement (180, 280, 380a, 380b, 580, 680) steuert, um Antriebsleistung auf die erste Antriebseinheit aufzubringen, um das Antriebsende (151, 251, 351a, 351b, 451a, 451b, 551a, 551b, 651a, 651b, 751a, 751b, 751c, 751d) anzutreiben, um die Radzähne (141, 241, 341, 441, 541a, 541b, 641, 741a, 741b) vorzuschieben, und steuert das Rückstellelement (170, 270, 370a, 370b, 570, 670), um Antriebsleistung auf die erste Antriebseinheit aufzubringen, um das Antriebsende (151, 251, 351a, 351b, 451a, 451b, 551a, 551b, 651a, 651b, 751a, 751b, 751c, 751d) zurückzustellen,
die Betriebsweise der ersten Antriebseinheit eine unidirektionale Bewegung oder eine Hin- und Herbewegung umfasst,
der Betriebsmodus der ersten Antriebseinheit die Amplitude der unidirektionalen Bewegung, die Amplitude der Hin- und Herbewegung oder die Bewegungsgeschwindigkeit umfasst, wodurch eine Vielzahl verschiedener Betriebsmodi der ersten Antriebseinheit unterschiedliche unidirektionale Bewegungen oder Hin- und Herbewegungen oder verschiedene Bewegungsgeschwindigkeiten umfassen,
das Leistungsmodul (980) unterschiedliche Kräfte in linearen Richtungen auf die erste Antriebseinheit aufbringt,
die erste Leistungseinheit (780, 880, 1080) einen elektrisch angetriebenen Linearaktuator oder einen elektrisch beheizten Linearaktuator umfasst, und die zweite Leistungseinheit (770, 870, 1070) einen elektrisch angetriebenen Linearaktuator, einen elektrisch beheizten Linearaktuator oder ein elastisches Element umfasst, und das Steuermodul (101) durch Steuerung der Größe oder der Ausgangsfrequenz der von der ersten Leistungseinheit (780, 880, 1080) oder der zweiten Leistungseinheit (770, 870, 1070) ausgegebenen Antriebsleistung die Amplitude der unidirektionalen Bewegung, die Amplitude der Hin- und Herbewegung oder die Bewegungsgeschwindigkeit der ersten Antriebseinheit steuert,
das mindestens eine Antriebsende (151, 251, 351a, 351b, 451a, 451b, 551a, 551b, 651a, 651b, 751a, 751b, 751c, 751d) mindestens zwei Antriebsenden (151, 251, 351a, 351b, 451a, 451b, 551a, 551b, 651a, 651b, 751a, 751b, 751c, 751d) umfasst und das mindestens eine Antriebsrad (140, 240, 340, 440, 540a, 540b, 640, 740a, 740b) zwei fest miteinander verbundene Antriebsräder (140, 240, 340, 440, 540a, 540b, 640, 740a, 740b) umfasst, und jedes Antriebsrad (140, 240, 340, 440, 540a, 540b, 640, 740a, 740b) mit mindestens einem der Antriebsenden (151, 251, 351a, 351b, 451a, 451b, 551a, 551b, 651a, 651b, 751a, 751b, 751c, 751d) zusammenwirkt.

2. Arzneimittelinfusionsvorrichtung mit mehreren Infusionsmodi nach Anspruch 1, **dadurch gekennzeichnet, dass**
das Antriebsrad (140, 240, 340, 440, 540a, 540b, 640, 740a, 740b) ein Ratschenrad ist und die Radzähne (141, 241, 341, 441, 541a, 541b, 641, 741a, 741b) Ratschenzähne sind.

3. Arzneimittelinfusionsvorrichtung mit mehreren Infusionsmodi nach Anspruch 2, **dadurch gekennzeichnet, dass**
das Antriebsende (151, 251, 351a, 351b, 451a, 451b, 551a, 551b, 651a, 651b, 751a, 751b, 751c, 751d) eine linear hin- und herbewegte Klinke (850) umfasst, die die Ratschenzähne schiebt, um die Ratsche intermittierend zu drehen.

4. Arzneimittelinfusionsvorrichtung mit mehreren Infusionsmodi nach Anspruch 1, **dadurch gekennzeichnet, dass**
die erste Antriebseinheit zwischen zwei Antriebsrädern (140, 240, 340, 440, 540a, 540b, 640, 740a, 740b) angeordnet ist und im Betrieb die erste Leistungseinheit (780, 880, 1080) und die zweite Leistungseinheit (770, 870, 1070) abwechselnd Antriebsleistung auf die erste Antriebseinheit aufbringen, wodurch die erste Antriebseinheit in den zwei Richtungen ihrer Hin- und Herdrehung unterschiedliche Antriebsräder (140, 240, 340, 440, 540a, 540b, 640, 740a, 740b) intermittierend dreht.

5. Arzneimittelinfusionsvorrichtung mit mehreren Infusionsmodi nach Anspruch 1 oder Anspruch 4, **dadurch gekennzeichnet, dass**,
wenn ein Antriebsrad (140, 240, 340, 440, 540a, 540b, 640, 740a, 740b) mit zwei Antriebsenden (151, 251, 351a, 351b, 451a, 451b, 551a, 551b, 651a, 651b, 751a, 751b, 751c, 751d) zusammenwirkt, die vorderen Enden der zwei Antriebsenden (151, 251, 351a, 351b, 451a, 451b, 551a, 551b, 651a, 651b, 751a, 751b, 751c, 751d) nicht auf gleicher Höhe liegen, wodurch die beiden Antriebsenden (151, 251, 351a, 351b, 451a, 451b, 551a, 551b, 651a, 651b, 751a, 751b, 751c, 751d) die Radzähne (141, 241, 341, 441, 541a, 541b, 641, 741a, 741b) asynchron schieben.

6. Arzneimittelinfusionsvorrichtung mit mehreren Infusionsmodi nach Anspruch 1, **dadurch gekennzeichnet, dass**
das Antriebsmodul ferner mindestens eine Hilfsantriebseinheit (1000) umfasst, die mit der ersten Antriebseinheit oder der zweiten Antriebseinheit verbunden ist und mit dieser zusammenwirkt, und die erste Antriebseinheit und die zweite Antriebseinheit über die Hilfsantriebseinheit (1000) miteinander zusammenwirken.

7. Arzneimittelinfusionsvorrichtung mit mehreren Infusionsmodi nach Anspruch 1, **dadurch gekennzeichnet, dass**
sie ferner eine Basis (190) umfasst, auf der die zweite Antriebseinheit beweglich montiert ist, und die Basis (190) und die zweite Antriebseinheit in Reibschluss stehen.

8. Arzneimittelinfusionsvorrichtung mit mehreren Infusionsmodi nach Anspruch 7, **dadurch gekennzeichnet, dass**
sie ferner ein positionsbegrenzendes Element (191) umfasst, das beweglich an der Basis (190) montiert ist, um die Position der zweiten Antriebseinheit zu begrenzen, und das positionsbegrenzende Element (191) und die zweite Antriebseinheit in Reibschluss stehen.

## Revendications

1. Dispositif de perfusion de médicaments à modes de perfusion multiples, comprenant:
un réservoir (110), un piston (120) et une vis (130), le piston (120), relié à la vis (130), étant disposé dans le réservoir (110) ;
un module d'entraînement, utilisé pour entraîner la vis (130) vers l'avant, comprend au moins une première unité d'entraînement (850, 950) et une deuxième unité d'entraînement qui coopèrent et sont reliées l'une à l'autre, la deuxième unité d'entraînement étant reliée à la vis (130) pour entraîner la vis (130) vers l'avant ;
un module d'alimentation (980) relié à la première unité d'entraînement ; et
un module de commande (101), relié au module d'alimentation (980), commande
le module d'alimentation (980) pour appliquer différentes puissances d'entraînement à
la première unité d'entraînement, ce qui permet à la première unité d'entraînement d'avoir une variété de modes de fonctionnement différents, **caractérisé en ce que**,
le module d'alimentation (980) comprend une première unité d'alimentation (780, 880, 1080) et une deuxième unité d'alimentation (770, 870, 1070) respectivement connectées à la première unité d'entraînement,
la première unité d'entraînement comprend au moins un élément d'entraînement (150, 250, 350a, 350b, 450a, 450b, 550, 650, 750) comportant au moins un arbre rotatif (160, 260, 360, 460a, 460b, 560) et au moins une extrémité d'entraînement (151, 251, 351a, 351b, 451a, 451b, 551a, 551b, 651a, 651b, 751a, 751b, 751c, 751d) tournant le long dudit au moins un arbre rotatif (160, 260, 360, 460a, 460b, 560), et
la deuxième unité d'entraînement comprend au moins une roue d'entraînement (140, 240, 340, 440, 540a, 540b, 640, 740a, 740b) pourvue de dents de roue (141, 241, 341, 441, 541a, 541b, 641, 741a, 741b), et l'extrémité d'entraînement (151, 251, 351a, 351b, 451a, 451b, 551a, 551b, 651a, 651b, 751a, 751b, 751c, 751d) pousse les dents de roue (141, 241, 341, 441, 541a, 541b, 641, 741a, 741b) vers l'avant pour faire tourner la roue d'entraînement (140, 240, 340, 440, 540a, 540b, 640, 740a, 740b), de manière à entraîner la vis (130) vers l'avant, amenant ainsi le dispositif de perfusion à fournir une perfusion de médicament,
la première unité d'alimentation (780, 880, 1080) est un élément d'avance (180, 280, 380a, 380b, 580, 680), tandis que la deuxième unité d'alimentation (770, 870, 1070) est un élément de réinitialisation (170, 270, 370a, 370b, 570, 670), et pendant le fonctionnement, le module de commande (101) commande l'élément d'avance (180, 280, 380a, 380b, 580, 680) pour appliquer une puissance d'entraînement à la première unité d'entraînement afin d'entraîner l'extrémité d'entraînement (151, 251, 351a, 351b, 451a, 451b, 551a, 551b, 651a, 651b, 751a, 751b, 751c, 751d) afin de faire avancer les dents de roue (141, 241, 341, 441, 541a, 541b, 641, 741a, 741b), et commande l'élément de réinitialisation (170, 270, 370a, 370b, 570, 670) pour appliquer une puissance d'entraînement à la première unité d'entraînement afin de réinitialiser l'extrémité d'entraînement (151, 251, 351a, 351b, 451a, 451b, 551a, 551b, 651a, 651b, 751a, 751b, 751c, 751d),
le mode de fonctionnement de la première unité d'entraînement comprend un mouvement unidirectionnel ou un mouvement de va-et-vient,
le mode de fonctionnement de la première unité d'entraînement comprend l'amplitude du mouvement unidirectionnel, l'amplitude du mouvement de va-et-vient ou la vitesse de déplacement ; par conséquent, divers modes de fonctionnement de la première unité d'entraînement comprennent différents mouvements unidirectionnels ou de va-et-vient, ou diverses vitesses de déplacement,
le module d'alimentation (980) applique différentes forces dans des directions linéaires à la première unité d'entraînement,
la première unité d'alimentation (780, 880, 1080) comprend un actionneur linéaire à entraînement électrique ou un actionneur linéaire à chauffage électrique, et la deuxième unité d'alimentation (770, 870, 1070) comprend un actionneur linéaire à entraînement électrique, un actionneur linéaire à chauffage électrique ou un élément élastique, et le module de commande (101), en contrôlant l'amplitude ou la fréquence de sortie de la puissance d'entraînement fournie par la première unité d'alimentation (780, 880, 1080) ou de la deuxième unité d'alimentation (770, 870, 1070), commande l'amplitude du mouvement unidirectionnel, l'amplitude du mouvement de va-et-vient ou la vitesse de déplacement de la première unité d'entraînement,
l'au moins une extrémité d'entraînement (151, 251, 351a, 351b, 451a, 451b, 551a, 551b, 651a, 651b, 751a, 751b, 751c, 751d) comprend au moins deux extrémités d'entraînement (151, 251, 351a, 351b, 451a, 451b, 551a, 551b, 651a, 651b, 751a, 751b, 751c, 751d), et ladite au moins une roue d'entraînement (140, 240, 340, 440, 540a, 540b, 640, 740a, 740b) comprend deux roues motrices reliées de manière fixe (140, 240, 340, 440, 540a, 540b, 640, 740a, 740b), et chaque roue d'entraînement (140, 240, 340, 440, 540a, 540b, 640, 740a, 740b) coopère avec au moins l'une des extrémités d'entraînement (151, 251, 351a, 351b, 451a, 451b, 551a, 551b, 651a, 651b, 751a, 751b, 751c, 751d).

2. Dispositif de perfusion de médicaments à modes de perfusion multiples selon la revendication 1, **caractérisé en ce que**,
la roue d'entraînement (140, 240, 340, 440, 540a, 540b, 640, 740a, 740b) est une roue à cliquet et les dents de la roue (141, 241, 341, 441, 541a, 541b, 641, 741a, 741b) sont des dents de cliquet.

3. Dispositif de perfusion de médicaments à modes de perfusion multiples selon la revendication 2, **caractérisé en ce que**,
l'extrémité d'entraînement (151, 251, 351a, 351b, 451a, 451b, 551a, 551b, 651a, 651b, 751a, 751b, 751c, 751d) comprend un cliquet à mouvement linéaire alternatif (850) qui pousse les dents de cliquet pour faire tourner le cliquet par intermittence.

4. Dispositif de perfusion de médicaments à modes de perfusion multiples selon la revendication 1, **caractérisé en ce que**,
la première unité d'entraînement est disposée entre deux roues d'entraînement (140, 240, 340, 440, 540a, 540b, 640, 740a, 740b), et pendant le fonctionnement, la première unité d'alimentation (780, 880, 1080) et la deuxième unité d'alimentation (770, 870, 1070) appliquent alternativement une puissance d'entraînement à la première unité d'entraînement, ce qui amène la première unité d'entraînement, dans les deux sens de sa rotation alternative, à entraîner différentes roues d'entraînement (140, 240, 340, 440, 540a, 540b, 640, 740a, 740b) à tourner par intermittence.

5. Dispositif de perfusion de médicaments à modes de perfusion multiples selon la revendication 1 ou la revendication 4, **caractérisé en ce que**,
lorsqu'une roue d'entraînement (140, 240, 340, 440, 540a, 540b, 640, 740a, 740b) coopère avec deux extrémités d'entraînement (151, 251, 351a, 351b, 451a, 451b, 551a, 551b, 651a, 651b, 751a, 751b, 751c, 751d), les extrémités avant des deux extrémités d'entraînement (151, 251, 351a, 351b, 451a, 451b, 551a, 551b, 651a, 651b, 751a, 751b, 751c, 751d) ne sont pas au même niveau, ce qui fait que les deux extrémités d'entraînements (151, 251, 351a, 351b, 451a, 451b, 551a, 551b, 651a, 651b, 751a, 751b, 751c, 751d) poussent les dents de roue (141, 241, 341, 441, 541a, 541b, 641, 741a, 741b) de manière asynchrone.

6. Dispositif de perfusion de médicaments à modes de perfusion multiples selon la revendication 1, **caractérisé en ce que**,
le module d'entraînement comprend en outre au moins une unité d'entraînement auxiliaire (1000) qui est reliée à la première unité d'entraînement ou à la deuxième unité d'entraînement et coopère avec celles-ci, et la première unité d'entraînement et la deuxième unité d'entraînement coopèrent l'une avec l'autre par l'intermédiaire de l'unité d'entraînement auxiliaire (1000).

7. Dispositif de perfusion de médicaments à modes de perfusion multiples selon la revendication 1, **caractérisé en ce que**
il comprend en outre une base (190) sur laquelle la deuxième unité d'entraînement est montée de manière mobile, et la base (190) et la deuxième unité d'entraînement sont assemblées par friction.

8. Dispositif de perfusion de médicaments à modes de perfusion multiples selon la revendication 7, **caractérisé en ce que**
il comprend en outre un élément de limitation de position (191) qui est monté de manière mobile sur la base (190) pour limiter la position de la deuxième unité d'entraînement, et l'élément de limitation de position (191) et la deuxième unité d'entraînement sont assemblés par friction.
